(19) Europäisches Patentamt
European Patent Office
Office européen des brevets

(11) **EP 3 553 503 B1**

(12) **EUROPÄISCHE PATENTSCHRIFT**

(45) Veröffentlichungstag und Bekanntmachung des Hinweises auf die Patenterteilung:
**17.07.2024 Patentblatt 2024/29**

(21) Anmeldenummer: **19000128.9**

(22) Anmeldetag: **14.03.2019**

(51) Internationale Patentklassifikation (IPC):
*G01N 21/84* (2006.01)  *G01N 21/94* (2006.01)
*A61L 2/28* (2006.01)  *B08B 13/00* (2006.01)
*A61B 90/70* (2016.01)

(52) Gemeinsame Patentklassifikation (CPC):
**G01N 21/94; A61L 2/28; B08B 13/00;**
**G01N 21/8483;** A61B 2090/702

(54) **ANALYSEVERFAHREN UND ANSCHMUTZUNG**

ANALYSIS METHOD AND SOILING

PROCÉDÉ D'ANALYSE ET SOUILLURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.04.2018 DE 102018002850**

(43) Veröffentlichungstag der Anmeldung:
**16.10.2019 Patentblatt 2019/42**

(73) Patentinhaber: **Simmoteit, Robert**
**72414 Rangendingen (DE)**

(72) Erfinder:
• **Simmoteit, Robert**
**72414 Rangendingen (DE)**
• **Simmoteit, Kim-Louis**
**69221 Rangendingen (DE)**
• **Simmoteit, Ken-Joel**
**76137 Karlsruhe (DE)**

(56) Entgegenhaltungen:
EP-A1- 3 231 447   EP-A2- 1 970 698
WO-A1-2015/193664   DE-A1- 102010 033 016
DE-A1- 102013 218 448   JP-A- 2010 220 970
US-A1- 2010 217 620

**Beschreibung**

Gebiet der Erfindung

Analyseverfahren für die optische Bestimmung von Teststreifen- oder von Kontrollflächen nach einer erfolgten Behandlung zur Bewertung der Reinigungs- oder der Sterilisationsleistung. Einsetzbar für unterschiedliche Teststreifen im Bereich der medizinischen Instrumentenaufbereitung sowie im Labor und in der Industrie.

Hintergrund der Erfindung

Für die Reinigung von medizinischen Hohlraumprodukten in Reinigungs- und Desinfektionsmaschinen kommen Testsysteme zum Einsatz, die auf der Oberfläche Blutanschmutzungen oder künstlich hergestellte Anschmutzungen aufweisen. Die Abreicherung dieser Anschmutzungen erlaubt derzeit nur eine Sichtanalyse oder eine Analyse der Restproteinmenge mit der OPA-Methode.

Analysemethoden für Teststreifen sind hinreichend bekannt und optische Analysemethoden gehören zum Stand der Technik. In EP 1 970 698 A2 wird eine Analysemethode beschrieben, in der Teststreifen mit einer Scanner-Technologie analysiert werden. Weiter sind Teststreifen in DE 10 2010 033 016.7 bekannt, deren Restanschmutzung mit einem optischen Verfahren ermittelt werden kann. Darüber hinaus wird in DE 10 2011 108 029.9 eine Prüfvorrichtung und Analyseverfahren beschrieben, bei der Teststreifen in einem Spaltkörper eingesetzt werden und anschließend die Reduzierung der Anschmutzungsfläche über eine Bildflächenanalyse bestimmt wird. In DE 10 2013 218 448 A1 wiederum wird eine Methode zur Bestimmung der Reinigungsleistung eingesetzt, bei der eine Proteinabreicherung auf einer Testfläche vorzugsweise quantitativ untersucht wird. Im Bereich der Sterilisationsleistung werden Testpapiere (z. B. Bowie Dick Testpapiere) eingesetzt. Die visuellen Analysen der Farbänderungen auf diesen Papieren ist dabei ein Maß für den Sterilisationserfolg. In WO2015 193 664 A1 wird hierzu eine Methode beschrieben, bei der ein Farb-Code eingesetzt wird, der sich bei der Reinigung und/oder Sterilisierung ändert und auslesbar ist. Weiterhin gehören wie in US 2010 0217 620 A1 elektronische Systeme im Bereich der Sterilisationsüberwachung zum Stand der Technik die einen auf einen Strichcode basierte Überwachungssystem und deren Spektren auslesen können.

Nachteilig ist jedoch, dass die Analysemethode immer auf den jeweiligen Teststreifentyp exakt abgestimmt ist und bei der visuellen Methode ohne Hilfsmittel keine genaue Analyse möglich ist. Bei den Analysen werden nicht allgemein verfügbare Analysegeräte eingesetzt, wodurch die Nutzung verteuert und erschwert wird.

Zusammenfassung der Erfindung

Die Erfindung hat die Aufgabe, die bereits bekannten Teststreifenanalysemethoden zu verbessern und durch die Nutzung von bekannten Handys, Scannern oder Kammers der Allgemeinheit zugänglich zu machen oder zumindest zu vereinfachen. Ziel ist ferner mit einem Analyseverfahren unterschiedliche Teststreifen oder Diagnostikträger, auch von bereits bekannten Testsystemen, auswerten zu können. Dabei ist vorgesehen unterschiedliche Teststreifengrößen oder Teststreifenformen und/oder Analyseparameter nach der Reinigung oder einer erfolgten Sterilisation zu untersuchen.

Diese Aufgabe wird erfindungsgemäß durch ein Analyseverfahren für die optische Bestimmung von Teststreifen- oder von Kontrollflächen nach einer erfolgten Behandlung zur Bewertung der Reinigungs- oder der Sterilisationsleistung nach Anspruch 1 gelöst. Folgende Ansprüche betreffen die vorteilhaften Ausgestaltungen der Erfindung.

Die Neuerung löst die Aufgabe durch ein Analyseverfahren, für die optische Bestimmung von Teststreifen-oder von Kontrollflächen nach einer erfolgten Behandlung mit einem Computerprogramm, wobei zumindest nach einer Reinigungs- und/oder Sterilisation der Teststreifen- oder der Kontrollflächen die noch vorhandenen Stoffe auf den Oberflächen der Teststreifen oder der Kontrollflächen im ersten Schritt mit optischen Methoden aufgenommen werden und es folgt eine Datenübermittlung an einem Server über das Internet und das Softwareprogramm zur Pixelanalyse auf dem Server nimmt zumindest eine Pixelzählung vor und bewertet die Pixel nach Intensität, um im zweiten Schritt mit Unterstützung von Markern, Zeichen, Rahmen, Barcodes oder Flächen die Pixel vor der Behandlung zu berechnen oder zu rekonstruieren oder diese virtuell zu erzeugen oder zu konstruieren, um in einem dritten Schritt zumindest durch die Abnahme der Pixelmenge oder durch die Pixeländerung einer Oberflächenfarbe und/oder eines Oberflächenmusters nach einer erfolgten Behandlung den Reinigungserfolg oder den Sterilisationserfolg einzelner Analyseergebnisse im Vergleich zu einem gesammelten Datenpool zu ermitteln In Weiterführung des Verfahren erfolgt die Auswertung gefärbter Flächen oder und/oder geometrischer Farbmuster oder bekannter Farbbildmuster mit Daten, die dem Computerprogramm bekannt sind oder mit Hilfe zumindest von Markierungen, Kennzeichnungen und/oder Eichbildern oder Eichflächen auf den Test- oder Kontrollflächen berechnet, rekonstruiert oder Eichbildern oder Eichflächen auf den Test- oder Kontrollflächen berechnet, rekonstruiert oder virtuell erzeugt werden, um mit diesen Daten die aufgenommenen Pixeldaten nach einer Behandlung zu vergleichen.

In einer weiteren Ausgestaltung des Analyseverfahren werden Teile oder der gesamte Datenpool oder die Auswertung oder die Interpretation der ermittelten Bildpunkte (Pixel) auf der Basis eines Maschinen lernenden Verfahrens der Informationstechnik (IT) bewertet (hierzu gehören z. B. Deep Learning Verfahren und statistisch basierte Modelrechnungen). Die Analyseergebnisse können dabei für sich alleine und/oder unter Bezugnahme einer größeren Datenmenge mehrere Nutzer zumindest statistisch bewertet und ausgewertet werden.

Im Bereich der Reinigungsanalytik, insbesondere für die Bestimmung von Anschmutzungsmengen auf Teststreifen- oder Kontrollflächen zur Bewertung der Reinigungsleistung, eignet sich das Verfahren dadurch, dass zumindest nach der Reinigung die Teststreifen oder Kontrollflächen Markierungen oder Kennzeichnungen besitzen und diese ungeordnet oder verstreut liegend mit optischen Methoden oder mit Bildaufnahmegeräten aufgenommen werden, um mit Hilfe der Markierungen oder Kennzeichnungen die Lage der Teststreifen oder der Kontrollflächen und die Anschmutzungsflächen mit einem Computer - oder Softwareprogramm (hier auch allgemein als Programm definiert) zu ermitteln und anschließend in den berechneten Anschmutzungsflächen zumindest die noch farbigen und auflösbaren Pixel zu analysieren, wobei ein Pixel näherungsweise eine Stoffmenge und/oder eine Farbmenge oder zumindest eine Farbintensität auf einer Oberfläche nach einer erfolgten Reinigung charakterisiert und hierrüber die Reinigungsleistung bestimmt wird.

Im ersten Schritt des Analyseverfahrens werden dabei die nicht mehr sichtbaren Testanschmutzungsflächen oder deren Flächenteile und deren Lage auf Teststreifen- oder Kontrollflächen untersucht. Hierzu werden Positionsmarkern oder Markierungen oder noch ein sichtbarer Testflächenteil oder mit einem Hintergrund der andersfarbig als die Teststreifenfläche ist, die Teststreifen aufgenommen und analysiert, um aus diesen Daten mit einem Computerprogramm die genutzte Testanschmutzungsfläche vor der Reinigung zu berechnen oder zu rekonstruieren. Dabei kann die geometrische Ausgestaltung der Teststreifen- oder der Kontrollflächen selbst (eckig, rund oder anders) eine wichtige Basisgröße für die Berechnung der technisch nutzbaren Anschmutzungs- oder Kontrollfläche sein.

In Weiterbildung des Verfahrens ist dadurch gekennzeichnet, dass bei der Auswertung der Flächen oder des geometrischen Farbmusters oder der Farbbilder die Flächenbereiche in der eine Farbänderung auf der Fläche nach der Reinigung oder nach der Sterilisierung oder nach einer Dekontamination erfolgt, bewertet, herausgehoben oder anzeigt werden, um damit zumindest eine Temperaturbehandlung und/oder eine pH-Wertänderung anzuzeigen und/oder um Partikel oder Reststoffe auf einer Fläche zu bestimmen. Hier kann mit Hilfe der Informationstechnologie (IT) und der eingesetzten Verfahren Flächenbereiche (Aktionsflächen) berechnen und dadurch sichtbar herausgehoben und/oder in Zonen einteilt werden, in denen Änderungen erfolgt sind.

Um die abgereicherte Testanschmutzungsfläche (diese kann durch die Reinigung fast vollständig entfernt worden sein) und deren Lage nach der Reinigung zu ermitteln, werden reinigungsstabile Markierungsstriche, Rahmungen, Punkte, Dreiecke oder Barcodes eingesetzt. Diese werden herangezogen, um zumindest über deren Breite oder Abstand zueinander sowie deren Größe mit einem optischen Verfahren nach der Reinigung bzw. nach der Abreicherung die zuvor vorhandene Anschmutzung- bzw. Kontaminationsfläche und/oder das Flächendesign und deren Ausrichtung auf dem Teststreifen über ein Computerprogramm zu ermitteln. Ferner wird die Analyse dadurch wirtschaftlich, weil die Teststreifen oder Kontrollflächen nicht bei der Aufnahme ausgerichtet werden müssen und unterschiedliche Teststreifenarten gleichzeitig untersucht werden können. Das Wissen um deren Größe und Anordnung ist notwendig, um einen Abreicherungsgrad oder eine Abreicherungsmenge nach der Reinigung ermitteln zu können, insbesondere um daraus die erbrachte Reinigungsleistung abzuleiten oder um das Areal einzugrenzen, in der sich die Anschmutzung vor der Reinigung befunden hat.

Weiter kann vorgesehen sein, dass die Markierungen fälschungssichere Informationen wie fortlaufende Barcodes, Nummerncodes oder Farbcodes oder Farbflächen besitzen. Diese lassen sich mit einem Softwareprogramm entschlüsseln. Beispielsweise kann vorgesehen sein, dass es jede Teststreifenmarkierung nur einmal gibt. Farbflächen z. B. können sich im Reinigungsprozess irreversibel verfärben. Dadurch wird verhindert, dass der Anwender den Teststreifen nicht manipulieren kann.

Durch das optische oder durch das Bildaufnahmeverfahren werden die Bildpunkte einer Restanschmutzung oder einer farbigen Marker- oder Markierungsfläche elektronisch erfasst und verrechnet. Im Falle eines Dekontaminations- oder Sterilisationsnachweises wird mit einem optischen Verfahren oder mit einem Bildaufnahmeverfahren die Farbstoffmenge ermittelt, die einen Farbumschlag erfahren hat. Es ist von Vorteil, wenn zuvor die Gesamtfarbstoffmenge oder die Gesamtfarbfläche bestimmt wurde. Die möglichen eingesetzten Bildaufzeichnungsgeräte können Scanner, Kameras oder Mikroskope sein. Darüber hinaus kann als zusätzlicher Parameter die Partikelmenge auf der definierten Teststreifenoberfläche bestimmt werden.

Die bevorzugten Teststreifenoberflächen besitzen einen weißen, roten blauen oder z. B einen grünen Hintergrund, je nach der Art der zu ermittelnden Farben oder sonstigen optisch erkennbaren Oberflächenerscheinungen. Es ist vorgesehen, dass ein Computerprogramm ein nicht sichtbares Flächenareal zumindest durch eine Größenangabe der vorhandenen Markierung auf dem Teststreifen berechnet, um im Anschluss die in dieser berechneten Fläche gefundenen Pixel oder Bildpunkte zu zählen. Dabei kann auch dem Computerprogramm im Vorfeld die Größe der Anschmutzungsfläche bekannt sein. Die Markierungen werden jedoch benötigt, um zumindest die Ausrichtung der Flächen festzulegen. Alternativ kann die Anschmutzungsfläche von einer Rahmung eingefasst sein, wobei die innere Anschmutzungsfläche aus geometrischen Formen oder ein Anschmutzungsraster besteht, deren Flächenteile mit einem Computerprogramm berechenbar sind. Die Änderung der inneren Farbstruktur dieser Anschmutzungsflächen z. B. bei der Reinigung oder sonstigen Behandlung kann ebenfalls zur Bewertung des Verfahrens herangezogen werden. Gemeint ist, dass z. B. Anschmutzungsflächen ein Schachbrettraster oder eine Wabenstruktur oder eine Farbabstufung aufweisen können und diese inneren Anschmutzungstrukturen nach der Reinigung sich verändert haben.

Die Markierungen können z. B. wie ein Barcode, ein Strich, eine Fläche, Punkte, Dreiecke oder ähnliches sein. Die Markierungsbreite oder die Markierungsfläche definiert dabei zumindest eine Analysefläche, die sich mittig, links oder rechts auf dem Teststreifen befindet. Über ein Computerprogramm werden die ermittelten Daten (Farbtiefe und Farbe der Pixel) berechnet und einer Flächengröße und Ausrichtung zugeordnet, welche wiederum durch die Markierung auf dem Teststreifen definiert ist. Dabei können ein, zwei oder mehrere Flächenareale durch unterschiedliche Markierungsarten bestimmt werden. Wesentlich ist dabei, dass die Markierungsbreite, die Markierungsfläche und/oder der Abstand zweier oder mehrerer Markierungen zur Berechnung und Orientierung der Analysenfläche herangezogen werden oder diese dadurch definiert ist. Die Markierungen (auch Einrahmungen) sollten durch die Reinigung nicht entfernt werden.

Eine Markierung, kann dabei auch selbst eine definierte Testanschmutzungsfläche sein, die geometrische Flächen (Dreiecke, Rechtecke oder ähnliches) besitzt, um über diese die Ausrichtung einer Testfläche anzugeben. Einfacher ist es, wenn die Anschmutzungsfläche zwischen zwei beabstandeten Markierungen oder vier Markierungspunkten oder links und rechts zu einer mittigen Markierung als Orientierung einer zu berechnenden Anschmutzungsfäche nutzbar ist.

Die Neuerung ermöglicht damit, dass unterschiedliche und mehrere Teststreifengrößen und - formen gleichzeitig z. B. auf einem Scannerglas bestimmt werden können. Dabei ist es nicht so wichtig, wie die Teststreifen auf dem Glas liegen, da durch die Markierungen Hilfsmittel vorhanden sind, um eine geeignete Flächenanalyse für jeden Teststreifen durchführen zu können. Befinden sich Barcodes (z. B. QR-Codes) als Markierungen auf den Teststreifen, so ist es ferner möglich aus den bereitgestellten Barcode Informationen mit einer Software herauszulesen. Die Software unternimmt dabei eine Pixelanalyse z. B. in der Art, dass die Pixelintensität, das Farbspektrum und deren Menge bestimmt wird. Diese Pixelanalyse ermöglicht zumindest physikalisch einen indirekten Stoffnachweis und/oder einen Stoffmengennachweis oder den Verlauf einer chemischen Reaktion über eine Farbveränderung. Hierfür ist es sinnvoll, die physikalischen Daten (zumindest Farbe und Farbtiefe der Bildpunkte) mit den real gefundenen Stoffmengen zumindest im Labor zu kalibrieren. Über die optische Analyse können zusätzliche Informationen, wie die bevorzugte Reinigungsrichtung oder die Reinigungsflächendarstellung nach einer Reinigung oder Flächen mit Arealen schlechter und guter Reinigung ermittelt werden. Das wiederum ist bei einer reinen chemischen Analyse nicht gegeben.

Das Einsatzgebiet der Neuerung ist in erster Linie die Restmengenanalyse nach der Reinigung in Reinigungs- und Desinfektionsautomaten. Darüber hinaus erlaubt die Analyse die Wiedergabe eines sich verändernden Farbmarkers auf einem Teststreifen nach der Sterilisierung oder nach einem Dekontaminationsschritt. Dieser Farbumschlag kann einen Behandlungsparameter wie z. B. eine Temperaturbehandlung oder einen pH-Wert einer Lösung anzeigen. Ferner können alternativ auch Partikel auf Filter bestimmt werden. Ferner erlaubt das Analyseverfahren auch die Bestimmung des Teststreifeneinsatzgebietes. Beispielsweise können unterschiedlich breite Teststreifen vorgesehen sein, um eine Innenlumenreinigung oder eine Außenflächenreinigung zu untersuchen. Im Falle der Innenlumen-Reinigungsanalyse fallen die Teststreifenbreiten sehr schmal (2- 6 mm) im Vergleich zu Teststreifen aus, mit denen Außenflächen (10 - 20 mm) analysiert werden. Die Markierung selber kann aber auch eine Referenzfläche ausbilden, um bei der optischen Analyse oder bei einer Bildaufnahme z. B. eine oder mehrere Farbflächen zur Eichung des Analysegerätes nutzen zu können.

Für die Analyse wird z. B. eine Software benötigt, die die erkannten Markierungen auf den Teststreifen nutzt, um hieraus eine Analysenfläche und deren Orientierung zu bestimmen. Dabei kann die Software Bestandteil einer Kamera, eines Mikroskops oder in einem Handy als App nutzbar sein oder auf einen Server zur Verfügung gestellt werden. Ferner kann weiter eine Zusatzbeleuchtung zur besseren Pixelbestimmung oder ein Teststreifen oder eine Kontrollfläche mit einer Musterfarbe oder mit einer Musterfarbtafel zur Eichung oder besseren Berechnung eingesetzt sein.

Es kann vorgesehen sein, dass die Software zumindest Bestandteil eines Analysegerätes ist oder über das Internet oder über Netzwerke genutzt wird. Die Softwarenutzung hat den Vorteil, dass die Analysen von Restverschmutzungen weltweit verfügbar sind. Das wiederum erlaubt eine Bewertung der bisher nutzbaren Reinigungsgeräte und der eingesetzten Reinigungschemie und verbessert zumindest die Standardisierbarkeit von Reinigungsprozessen.

Das Programm nutzt physikalische Parameter zur Bewertung der Pixel und ist in der Lage diese hinsichtlich der Farbintensität bzw. der Farbsättigung zu ordnen. Darüber hinaus sind der Farbton und die Helligkeit der ermittelten Bildpunkte oder die Flächenausgestaltung eines Pixels oder einer Teilfläche ein weiteres Kriterium der Bewertung. Ferner ist es möglich, die gefundenen Pixel auf einer Fläche sichtbar anzuordnen und gegeben falls zu verstärken (z. B. durch eine Mikroskopvergrößerung), um z. B. bei der Reinigungsanalyse noch so kleine Restkontaminationen sichtbar zu machen. Das eingesetzte Verfahren nutzt die Farbsättigung oder die Fluoreszenz z. B. einer roten Farbe für eine quantitative Analyse der noch vorhandenen Stoffmenge auf einer Testfläche. Die Farbintensität ist z. B. ein Ergebnis der Anzahl an farbigen Molekülen, die insbesondere auch übereinander gelagert, vorliegen können und ist damit ein Maßstab für eine filmische Anschmutzung auf einer Oberfläche. Wird die gemessene Farbintensität einer Stoffmenge auf einer Oberfläche in Bezug gesetzt, lässt sich alleine durch die Bestimmung der Farbintensität einer Farbe näherungsweise auf eine Stoffmenge zurückschließen, in der die Farbmoleküle eingebunden sind.

Das Analyseverfahren zeichnet sich insbesondere dadurch aus, dass ein optisch erfasster Pixel (Bildpunkt oder kleinstes Element einer digitalisierten Rastergraphik) näherungsweise eine realen Stoffmenge z. B. auf einer Oberfläche zuordenbar ist. Die im Pixel verwendete Kodierung einer Farbe definiert sich unter anderem über den Farbraum und die Farbtiefe die mit physikalisch Methoden (optischen Bildsensoren) erfassbar ist. Das Verfahren nutzt dabei bestimmbare Farbmoleküle die in Stoffmengen (Anschmutzungen) vorhanden sind. Insbesondere bei denen die Farbmoleküle vorzugsweise an Anschmutzungsmoleküle chemisch gebunden und/oder durch schwache nicht-kovalente Wechselwirkungen (van-der- Waals Wechselwirkung) festgehalten oder in die Anschmutzungsmatrix eingebettet sind. Bevorzugt ist dabei die Anschmutzung eine filmischen Beschichtungsmatrix auf einer Oberfläche. Über eine Eichung wird eine Stoffmenge mit den hier enthaltenden Farbmolekülen über optische Verfahren aufgenommen und die gewonnen Pixel (Bildpunkte) einer Stoffmenge zugeordnet. Die gewonnen Umrechnungszahlen zur Berechnung einer Stoffmenge werden eingesetzt, um über eine Pixelzählung die Stoffmenge zumindest näherungsweise mit einem dem Computerprogramm zu berechnen oder eine Stoffflächengröße zu rekonstruieren.

Die Umrechnungszahlen (Umrechnungsfaktor) sind durch Labormethoden zu ermitteln und stellen in der Regel eine akzeptable Näherung zur Berechnung der Stoffmengen dar. Hierbei ist von Vorteil, wenn geringe und filmisch vorliegende Restanschmutzungsmengen für diese Ermittlung herangezogen werden, um sicher zu stellen, dass nicht zu viele Farbmoleküle übereinander liegen. Ist dem Computerprogramm dieser Bezug Farbintensität und Stoffmenge durch Realmessungen unter Laborbedingungen bekannt (also die Kennzahlen für die Umrechnung), kann über die ermittelte Farbintensität auf einer Oberfläche eine Stoffmenge zumindest rechnerisch ermittelt werden.

Die Neuerung ist deshalb von Bedeutung, weil durch die Abreicherung einer Farbschicht sich dabei auch die Farbintensität oder die Farbtiefe dieser Farbschicht ändert. Das Ergebnis ist, dass die Anzahl der übereinander gelagerten Farbmoleküle reduziert und dadurch die Genauigkeit der Messung verbessert wird.

Ein Pixel kann ein Speicherplatz von drei Bytes (je eines für die Farbkomponenten rot, grün und blau) einnehmen und dabei kann 1 Byte = 255 Intensitäts-Schritten einer Farbe z. B. in einem RGB Farbraum darstellen, wobei die Farbintensität/Farbtiefe durch den Speicherplatz bestimmt wird, den ein Pixel belegt. Es können unterschiedliche Modelle zur Darstellung von Farbräumen Anwendung finden wie z. B. das RGB-, XYZ-, CMYK-, LAB- oder das LMS Farbraum-System, um nur einige zu nennen. Beispielsweise kann der Informationsgehalt eines Pixels 48 Bit = 6 Byte sein (also 2 Byte für jede Farbe). Ferner ist je nach Farbwahl und Beleuchtung ein Mittel zwischen Effizienz und Genauigkeit einzustellen. Für eine Pixel-Auswertung über einen Scanner oder einer Kamera bedeutet das, dass vorzugsweise zumindest 2 Bytes pro Farbpixel einen Speicherplatz belegen. Rein technisch kann ein Pixel auch nur einen Speicherplatz von 1 Byte belegen. Die Pixel-Menge eines optisch erfassbaren Stoffes auf einer Oberfläche ist ein sinnvoller Maßstab und Hinweis für eine real vorhandene Stoffmenge auf einer Oberfläche. Die Reduzierung einer Ausgangsfarbintensität nach einer Reinigung ist wiederum ein Maßstab für eine erfolgreiche Reinigung oder Stoffreduzierung auf einer Oberfläche und ermöglicht darüber hinaus eine Stoffmengenangabe einer Restanschmutzung ohne diese selber mit aufwendigen Labormethoden untersuchen zu müssen. Zur Ermittlung einer erfolgreichen Sterilisation, wäre hingegen die Farbmengenumwandlung eines Farbstoffes ein Maßstab für eine erfolgreiche Sterilisationsleistung.

Die mit einem geeigneten Softwareprogramm gewonnen Daten werden in einer Datenbank abgelegt und können weltweit über das Internet verschickt werden. Erfindungsgemäß erfolgt eine Datenübermittlung an einem Server über das Internet. Auf dem Server befindet sich das Softwareprogramm zur Pixelanalyse. Das Softwareprogramm nimmt zumindest eine Pixelzählung vor und bewertet die Pixel nach Intensität. Ferner kann vorgesehen sein, dass die Pixel auf einer Fläche angeordnet werden oder das Programm führt eine Flächenberechnung im Vergleich zu einer bekannten Gesamtflächengröße durch. Dabei kann weiter eine Datenbank eingesetzt sein, um die Analyse mit z. B. standardisierten oder kontrollierten Daten zu unterstützen und eine fehlerhafte Auswertung zu reduzieren.

Weiter kann vorgesehen sein, dass die Softwaredaten in einer Datenbank ablegt sind, um diese für weitere Analysen nutzbar zu machen. Beispielsweise können die hier aufgeführten Daten in Diagramme überführt werden, um für den Nutzer Vergleichs- und/oder Verlaufsdaten bereitzustellen. Alternativ vermag die Software die gewonnen Daten so zusammenzustellen, dass sich hieraus technische Entwicklungen bewerten lassen. Die computerunterstützte Bewertung weist erfindungsgemäß einen großen in der Praxis ermittelten Datenpool auf, um beispielsweise eine neue Spülmethode oder ein neues Spülhilfsmittel mit den Daten aus älteren bekannten Methoden oder Hilfsmitteln zu vergleichen.

Da die Reinigungsleistung erheblichen Schwankungen unterliegt, sollten größere Datenmengen erhoben werden, um einen Reinigungserfolg sichtbar zu machen oder bewerten zu können. Hier wiederum können Algorithmen oder algorithmische Formeln oder statistische Methoden und/oder Maschinen lernende Methoden (z. B. Deep Learning) zur besseren Auswertung herangezogen werden. Die mathematische Betrachtung von einer großen Anzahl an Reinigungsdaten ermöglicht ferner Daten miteinander zu kombinieren, Bezüge herzustellen oder wichtige Informationen zu ermitteln. Mit der Neuerung lassen sich nicht nur Informationen anders nutzen, sondern auch wichtige Fragen beantworten wie: Wie wirkt sich die Lage einer Anschmutzungsfläche auf die Reinigung aus oder welchen Anteil am Reinigungsergebnis hat die Reinigungschemie oder der Spüldruck? Mit der Neuerung kann somit ein Reinigungsergebnis umfassender und schneller bewertet werden als derzeit möglich.

Eine mögliche Berechnung, der mit einem Scanner ermittelten Pixel-Menge ermöglicht die folgende Formel:

$$\text{Pixel Rest (Restkontamination)} \times 100\% / \text{Pixel Gesamt (Gesamtkontamination)} = \text{Reinigungserfolg in } \%$$

Die Bewertung oder Darstellung der Ergebnisse kann sich z. B. wie folgt darstellen:

| berechneter Wert | Bewertung |
|---|---|
| kleiner 0,1 % | 100 % sauber |
| kleiner 0,5 % | akzeptierte Sauberkeit |
| kleiner 1 % | Warnlevel |
| größer 1 % | nicht tolerierte Sauberkeit |

Bei der obigen Betrachtung ist es sinnvoll, wenn Grenzwerte für einen sicheren Farbnachweis bzw. eine Farberkennung in die Berechnung einbezogen werden. Alternativ kann anstelle der prozentualen Berechnung hier auch ein Umrechnungsfaktor z. B. von Pixel in [μg] oder [ng] eingesetzt sein.

Es ist daher verständlich, dass eine softwareunterstützte Datenerhebung zur Bewertung geeigneter Teststreifen und Testanschmutzungen einen erheblichen Vorteil für die Reinigungsindustrie darstellt. Darüber hinaus erhält der Nutzer nachvollziehbare Daten über seinen Reinigungserfolg und ist nicht auf eine anfällige und individuelle Sichtanalyse angewiesen.

Der Datenaustausch über das Internet ermöglicht ferner die internationalen Reinigungs- und Sterilisationsstandards anzugleichen und durch große Datenerhebungen der gesamten Welt nutzbar zu machen. Darüber hinaus wird es so erst möglich, technische Entwicklungen zumindest im Reinigungsbereich effizienter und über eine großangelegte Datenbereitstellung zu bewerten. Die unzureichende und bisherige Sichtanalyse von medizinischen Instrumentenoberflächen nach der Reinigung gehört damit der Vergangenheit an.

Bei der Neuerung ist vorgesehen eine farbig filmische Anschmutzungsfläche oder filmische farbige Flächenareale einzusetzen, die aus einem Biostoffgemisch mit einem 2 - 12 % Emulgatoranteil oder aus geronnenem Blut oder eine Kombination aus diesen besteht. Das Auftragen der filmischen Anschmutzung erfolgt im Druckverfahren oder über 3D-Drucker, um zumindest eine gleichmäßig oder strukturierte Fläche herzustellen oder mehrere Schichten übereinander anzuordnen. Nach dem Trocknungsprozess ist der Teststreifen oder die Reinigungskontrollfläche einsetzbar. Das vorgeschlagene Analyseverfahren kann dabei ebenfalls eingesetzt werden, um die Beschichtungsqualität der hergestellten Teststreifen in der Fläche zu bewerten.

Als Anschmutzung wird vorzugsweise ein Biostoffgemisch eingesetzt, das aus Proteine und Lipide und/oder Zuckerverbindungen und Emulagatoren besteht. Mögliche einsetzbare Emulgatoren sind Lezithin, Phosphorsäure, Natriumphosphat, Algin oder Propylenglykolalginat, Polyoxyethylenstearat, Ammoniumphosphatide, Natriumsalze oder Mono- und Diglyceride von Speisefettsäuren, Essigsäure-, Milchsäure-, Zitronensäure-, Weinsäure- oder Diacetylweinsäuremonoglyceride, Zuckerester von Speisefettsäuren oder Zucker- oder Polyglyceride von Speisefettsäuren, Polyglycerin-Polyricinoleat, Propylenglykolester von Speisefettsäuren oder Stearinsäure. Als bevorzugter natürlicher Emulgator wird Lezithin, Cholesterin oder Alginat oder ein Gemisch aus diesen in einer Größenordnung z. B. von 2 -10 % eingesetzt. Das Stoffgemisch selber kann aus 5 - 35 % Lipide, 2 - 20 % Protein, 0,2 - 2 % Kohlenhydrate, 0,1 - 1 % Mineralstoffe und Farbstoffe, die mit Wasser und/oder mit Blut vermischt sind, bestehen. Im Falle von Blut kann noch zusätzlich Thrombin zur Förderung der Gerinnung eingesetzt sein.

Der Blutbestandteil der Anschmutzung kann dabei vollständig die Aufgabe eines Farbstoffs übernehmen. Von Vorteil ist es, wenn chemisch unbedenkliche Farbstoffe oder Farbpigmente eingesetzt werden und/oder biologische Stoffe, wie Blut oder Lebensmittelbestandteile z. B. aus dem Ei, aus Tomaten oder Rotkraut zur Färbung oder zur Erhöhung der Stabilität eingesetzt werden.

Die Emulsion wird vorzugsweise flächig im Druckverfahren aufgetragen und ergibt eine Kontaminations- oder Anschmutzungsfläche. Zur besseren Haftung sind diese Flächen angeraut oder besitzen eine definierte Oberflächenstrukturierung. Alternativ kann auch eine Haftvermittleroberfläche mit einem Plasmaverfahren hergestellt werden.

Damit die Neuerung wirtschaftlich einsetzbar ist, sind Teststreifen zu bevorzugen, deren Aufbau zumindest die chemische und physikalische Behandlung unbeschadet überstehen. Ferner sind die nutzbaren Teststreifengrößen so zu wählen, dass diese in Teststreifenhalterungen zumindest bei der Reinigung nutzbar sind.

Einsatzgebiete und Ausführungsformen

Der bevorzugte Aufbau der Teststreifen ist 2 bis 20 mm breit und 5 - 20 cm lang und im Falle von Kontrollflächen können diese rund, Quadratisch oder anders geformt sein. Der Teststreifen besitzt zumindest in der Mitte eine Anschmutzungsfläche bzw. eine Kontaminationsfläche die kleiner als die Gesamtteststreifenfläche ist. Die Anschmutzungsfläche kann unterschiedliche Marker und/oder biologische Proteine und/oder Fett aufweisen oder eine Kombination aus diesen beinhalten. Die einsetzbaren Teststreifen oder Kontrollflächen werden bevorzugt in Teststreifenhalterungen wie in DE 10 2010 033 016.7 beschrieben, eingesetzt. Dabei kann die Anschmutzungsfläche geometrische Muster aufweisen oder nach der Reinigung einen vor der Reinigung gesicherten Referenzpunkt oder eine Referenzfläche besitzen deren Ausrichtung nach der Reinigung eine Gesamtkontaminationsfläche definiert. Diese wird z. B. durch einen Stempel oder durch eine Schraube erzeugt, die während der Reinigung auf die Kontaminationsfläche gepresst wurde und diese Fläche vor der Reinigung schützt.

Als mögliche Auslesegeräte kommen käufliche Scanner zum Einsatz oder alternativ können mit einer Kamera oder einer Handy-Kamera Bilder von Teststreifen aufgenommen werden. Zur Standardisierung kommen dabei Musterteststeifen und/oder Farbkarten zum Einsatz, um die Beleuchtungsart oder Farbverschiebungen zu reduzieren. Weiter sind Beleuchtungshilfsmittel zur korrekten Beleuchtung der Teststreifen bei der Aufnahme denkbar. Darüber hinaus können auch Stative oder Bildabstandsvorrichtungen eingesetzt sein, um die Aufnahme zu standardisieren.

Um Teststeifen ohne Markierungen auslesen zu können, werden farbige Hintergründe oder Abdeckungen eingesetzt. Beispielsweise wird ein weißer Teststreifen einem blauen oder grünen Hintergrund ausgesetzt. Über eine Software werden die aufgenommenen Teststreifen und deren Gesamtgröße ermittelt und hieraus wiederum wird die zu analysierende Kontaminationsfläche berechnet, welche nach der Reinigung verschwunden ist und in der Regel so nur noch eine ganz geringe Restkontamination vorhanden sein kann.

Für die Ermittlung der Sterilisationsleistung können alle gängigen Bowie Dick Testpapiere oder Teststreifen eingesetzt werden. Das farbig markierte Papier wird dabei im Dampfsterilisator in einer genormten Vorrichtung eingesetzt. Nach der Sterilisation sollte der Farbmarker vollständig umgewandelt sein, was jedoch nicht immer der Fall ist. Hier wird das Analyseverfahren eingesetzt, um mit optischen empfindlichen Methoden der Bilderkennung eine genauere Analyse durchzuführen, als es per Auge möglich ist.

Die Teststreifen oder Kontrollflächen können z. B. aus Kunststoff oder aus Metall hergestellt sein und für folgende Untersuchungen eingesetzt werden:

1. Reinigung von Hohlrauminstrumenten aller Art.
2. Reinigung von Produktaußenflächen aller Art.
3. Reinigung in Ultraschallbäder oder in medizinischen Reinigungsautomaten.
4. Entfettung von Produkten
5. Abtragung von Oberflächenstrukturen
6. Ermittlung von Farbumschlägen, die einem pH-Wert oder einer Temperaturbehandlung zuordenbar sind.
7. Ermittlung der Sterilisationsleistung mit Hilfe von temperatursensiblen Farbmarkern oder Markierungsflächen.

Das bevorzugte Verfahren besteht aus einem Scanner, der einen farbigen Hintergrund im Deckel aufweist. Im ersten Schritt werden die Teststreifen auf dem Scannerglas abgelegt. Die Orientierung oder dessen Größe ist hierbei in der Regel unerheblich. Im zweiten Schritt erfolgt ein Scan und eine Größenermittlung der Teststreifen sowie die Ermittlung der Restkontamination auf den Streifen. Im dritten Schritt werden die Bildpunkte analysiert und falls vorhanden mit Daten in einer Datenbank verglichen und/oder die gemessenen Bildpunkte einer Qualitätsanalyse unterzogen, um Fehlmessungen oder eine Fehlinterpretation der Daten zu reduzieren.

Ein weiteres Verfahren besteht darin, dass eine Handy App oder mit einem Computer Programm eingesetzt wird, mit der bei der Handy-Kamera-Aufnahme Farb- und Beleuchtungsunterschiede ausgeglichen werden. Die über die App ermittelten Bilder werden über das Internet in einem Server verarbeitet und die berechneten Daten dem Nutzer übermittelt.

Weitere Vorteile der Erfindung ergeben sich aus der Wahl der eingesetzten Testmaterialien, den weiteren Beschreibungen und den beigefügten Zeichnungen. Desweiteren aber auch durch die vielen Möglichkeiten einer elektronischen Datenverarbeitung, der Darstellung und Aufbereitung der Daten.

Es versteht sich, dass die vorstehenden genannten und die nachstehenden noch zu erläuternden Merkmale nicht nur in der jeweils angegebenen Kombination, sondern zum Teil auch in anderen Kombinationen oder in Alleinstellungen verwendbar sind. Ausführungsbeispiele der Erfindung sind in den Zeichnungen dargestellt und werden in den nachfolgenden Beschreibungen näher erläutert.

Es zeigen:

Fig. 1.    Draufsicht eines Teststreifens mit einer Kontaminationsfläche vor und nach der Reinigung.
Fig. 2.    Restanschmutzungen und deren Ermittlung auf einer Oberfläche
Fig. 3.    Draufsicht von Teststreifen mit unterschiedlichen Markierungen.
Fig. 4.    Anordnung von unterschiedlich großen Teststreifen auf einem Scanner-Glas
Fig. 5.    Schematische Darstellung einer Analyse und Übertragung von Pixel-Daten
Fig. 6.    Testfläche zur Bestimmung der Sterilisationsleistung

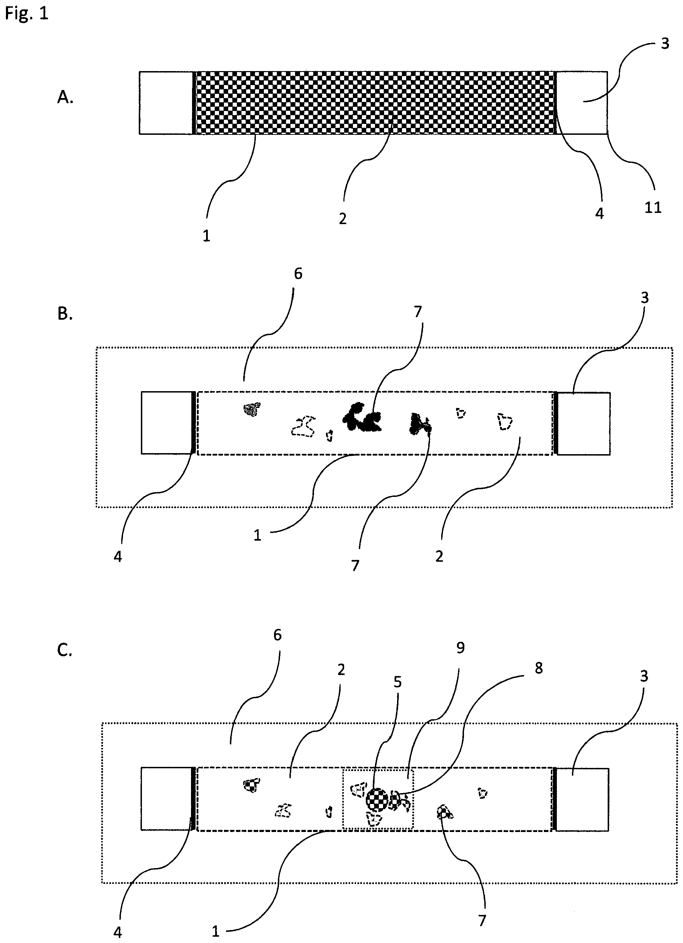

Figur 1. Zeigt in Draufsicht einen Teststreifen mit einer Kontaminationsfläche vor und nach der Reinigung. Die Abbildung **A.** zeigt einen Teststreifen **1** vor der Reinigung mit einer sichtbaren Anschmutzungsfläche **2**. Die Anschmutzungsfläche kann dabei ein Farbmuster aufweisen oder ganzfarbig sein. Links und rechts der farbigen Anschmutzungsfläche befinden sich Kennzeichnung als Strichte **4** und hinter diesen eine freie Teststreifenfläche **3**. Diese kann individuell beschriftet werden. Die vier Ecken **11** des Teststreifens oder die Striche **4** können nach der Reinigung herangezogen werden, die nicht mehr sichtbare Anschmutzungsfläche **2** und deren Orientierung mit einem Computerprogramm zu berechnen. Wird, wie in Abbildung **B,** ein andersfarbiger Hintergrund **6** als die Teststreifenfläche eingesetzt, so kann auch ohne die vorhandenen Kennzeichnungsstriche die eingesetzte Anschmutzungsfläche **2** nachträglich berechnet werden und die in dieser Fläche gefundenen Bildpunkte (Pixel) **7** sind Farbreste, die mit einem Scanner oder einer Kamera aufgenommen werden. Wichtig ist dabei die korrekte Orientierung der nicht mehr sichtbaren Anschmutzungsfläche **2** (Fläche auf die Stoffe aufgebracht sind), da diese den Bereich darstellt, in dem die Analyse stattfindet. Die sichtbaren Farbreste **7** werden in einem Softwareprogramm als Pixel aufgelöst und bestehen zumindest aus ein Protein- und/oder Lipidgemisch mit Zusatzstoffen. Der enthaltene Farbstoff oder die Farbstoffe lassen sich zwar physikalisch oder chemisch herauslösen, werden jedoch nur dann ganz entfernt, wenn auch die Matrix entfernt wurde. Auf Grund dieser Basis stellen die noch feststellbaren farbigen Farbreste Restanschmutzungen einen Reinigungsnachweis dar oder sind die Grundlage für eine Reinigungsleistungsberechnung, insbesondere dann, wenn die Gesamtanschmutzungsmenge bekannt ist. Die auf den Teststreifen aufgetragenen Schichten liegen im $\mu$m-Bereich und sind somit ultradünn, was die Sensitivität der Analysemethode erhöht. Ferner kann aus der Verteilung der Restanschmutzungen, deren Größe und deren Anzahl weiter Schlüsse auf den Reinigungserfolg gezogen werden. Der Teststreifen B kann in einem Hohlprüfkörper als Nachweis für die Innenlumenreinigung eingesetzt worden sein, oder alternativ auch als Nachweis nur für die Außenflächenreinigung in Verbindung mit einer Instrumentenoberfläche. Die Abbildung C zeigt, wie die Abbildung B, einen Teststreifen nach erfolgter Reinigung. Dieser wurde jedoch in einem Prüfkörper eingebracht der von oben einen Stift oder ähnliches auf die Anschmutzungsfläche gedrückt hat. Der vor einer Reinigung geschützte Anschmutzungsflächenbereich **5** bleibt nach der Reinigung erhalten. Da um den geschützten Bereich **9** ein schwer zugänglicher Raum oder Spalt angeordnet sein kann, werden hier mehr Restanschmutzungsflächen in Form von Pixel **7** gefunden als links und rechts von diesem geschützten Bereich **9**. Die Größe und Anordnung der gestrichelten Flächen **2** und **9** wurden über ein Computerprogramm berechnet. Zur Berechnung wurden die beabstandeten Kennzeichnung in Gestalt von Stichen **4** herangezogen.

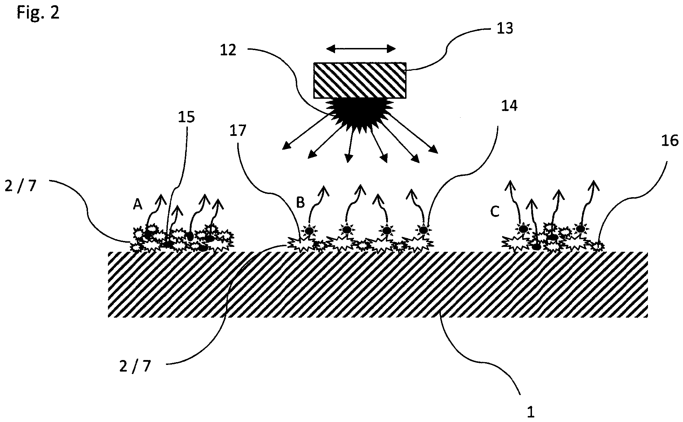

Figur 2. zeigt in Detailansicht Restanschmutzungen und deren Ermittlung auf einer Oberfläche. Mit einem Scanner oder mit einer optischen Methode wird ein Teststreifen **1** von oben bzw. von unten mit einer Lampe **12** beleuchtet und mit einen bewegbaren Abtastarm **13** abgetastet (dargestellt durch einen Pfeil). Die von den eingelagerten Farbmolekülen **15** oder von den Farbkomplexen **14** reflektierten Strahlen werden z. B. von Scanner-Sensoren/Halbleiter aufgefangen, um so Stromstärken zu erzeugen, die dann in Binärcodes umgewandelt werden, um daraus Bildpunkte oder ein Bild zu erzeugen. Die generierten Pixel beinhalten so verarbeitbare Informationen der eingebundenen Farbmoleküle in einer Anschmutzungsmatrix **2** oder **7** auf einer Teststreifenoberfläche. Alternativ können direkt die Anschmutzungsmoleküle **16** über eine geeignete Lichtquelle angeregt werden oder es können hier Fluoreszenzfarbstoffe eingesetzt sein, um die Mess-Empfindlichkeit zu erhöhen. In **A** ist eine filmische Anschmutzungsmatrix (im $\mu$m Bereich) gezeigt, in der nur physikalisch (über Van-der-Waals-Kräfte) Farbstoffe oder Farbstoffgemische eingelagert sind. Der Oberflächenbereich **B** besitzt ausschließlich einen Farbstoff **14,** der fest mit einem Anschmutzungsmolekül **17** (Farkmolekülkomplex) verbunden ist und zusammen eine filmische Restanschmutzung **7** zeigt. Der Darstellungsbereich **C** zeigt ein Gemisch aus eingelagerten **15** und chemisch verbundenen Farbstoffen **14**. Es können dabei unterschiedliche Farbstoffe zum Einsatz kommen, um beispielsweise diese unterschiedlichen Anschmutzungsstoffen zuordnen zu können. Alternativ zu den sichtbaren Farbstoffen **14** und **15** können auch fluoreszierende Farbstoffe eingesetzt sein.

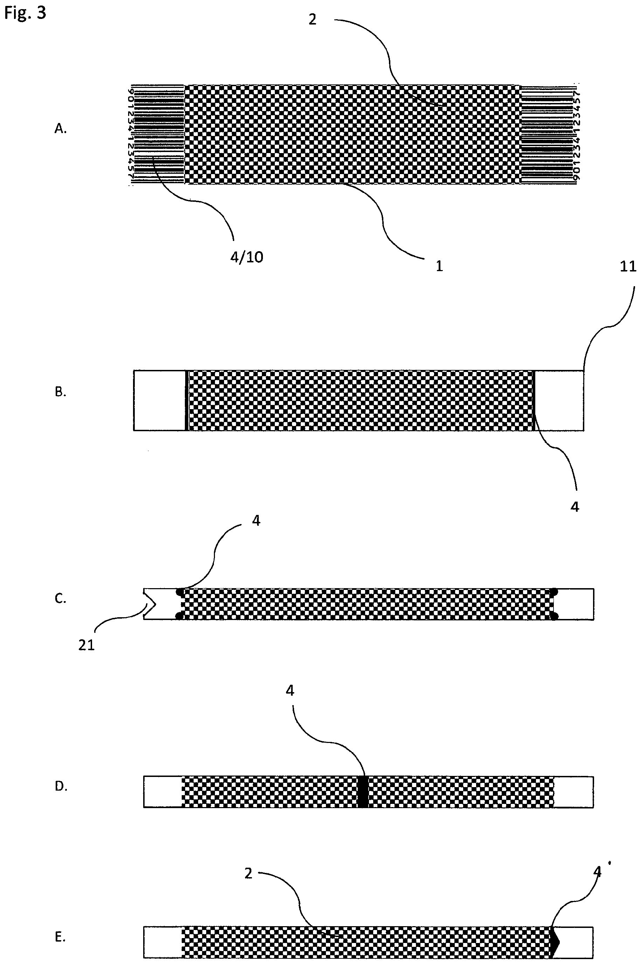

Figur 3. zeigt eine Draufsicht von Teststreifen mit unterschiedlichen Markierungen. Die Abbildung **A** zeigt einen Teststreifen **1** oder einen Kontrollkörper, der links und rechts auf einer anschmutzungsfreien Fläche als Kennzeichnung **4** einen Barcode **10** zur Wiederfindung oder zur Orientierung der Anschmutzungsfläche **2** nach erfolgter Reinigung besitzt. Die Kennzeichnung **4** kann dabei über einen Zahlencode **10** oder nur durch die Strichanordnung einmalig sein, also kein zweites Mal vorkommen. Damit kann der Barcode **10** auch nicht zweimal eingelesen werden, was Manipulationen verhindert und gleichzeitig ein Produktsicherheitnachweis ist. Abbildung B ist ebenfalls ein Teststreifen mit einer Anschmutzung, jedoch mit beabstandeten Strichkennzeichnungen **4**. In C befinden sich auf den Teststreifen beabstandete Punkte, die zusammen eine Anschmutzungsfläche einrahmen. Ferner kann eine Seite des Teststreifens eine Einkerbung **21** aufweisen, um eine Behandlungsrichtung zu dokumentieren. Bei der Darstellung **D** befindet sich mittig auf einer Anschmutzungsfläche eine Kennzeichnungsfläche **4**. Die Rechtseckige Anordnung ermöglicht an der Längsseite die Bestimmung der links und rechts vorhandenen Anschmutzungsflächen über das ungleiche Seitenlängenverhältnis der Kennzeichnungsfläche **4**. Die Abbildung **E** wiederum zeigt einen Teststreifen, der als Kennzeichnung nur ein Dreieck **4** besitzt. Über ein Dreieck **4** und deren Lage und Seitengrößen lässt sich die Ausrichtung und die Größe einer Anschmutzungsfläche bestimmen. Über die Anordnung eines Dreiecks kann man die Spülrichtungsseite, mit der der Teststreifen überspült wurde darstellen. Allen Kennzeichnungen **4, 10** ist gemein, dass diese durch das Reinigungsverfahren nicht entfernbar sind. Die Teststreifenfläche **1,** deren Geometrie oder Größe wird mit in die Berechnung einbezogen oder ist ein Berechnungsmaß.

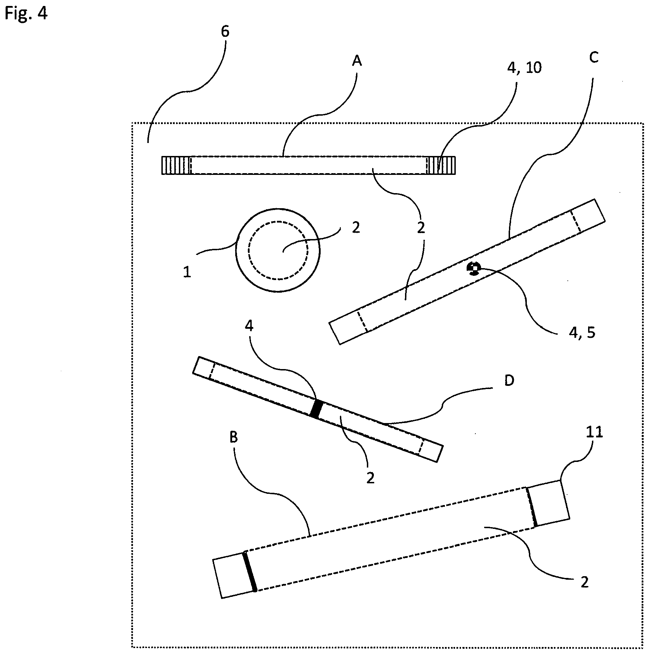

Figur 4. zeigt Anordnungen von unterschiedlich großen Teststreifen auf einem Scanner-Glas. Werden unterschiedlich Teststreifen oder Testkörper, dargestellt mit den Bezeichnungen **A-C,** auf einem Scannerglas **6** (alternativ farbiger Hintergrund) abgelegt, so ist es nicht wichtig, wie die Teststreifen auf dem Glas **6** liegen. Ferner ist hier auch eine runde Testfläche **1** gezeigt, die eine Runde Anschmutzungsfläche **2** oder Farbfläche besitzt. Alternativ kann **6** auch ein farbiger Hintergrund sein und die Aufnahme mit einer Kamera erfolgen. Durch die Kennzeichnung **4, 5** auf den Teststreifen oder durch die Flächenecken **11** oder durch die Flächenform **1** (rund, quadratisch, Eckig usw.) selber, kann die nicht mehr vorhandene Gesamtanschmutzungsfläche **2** mit Hilfe eines Computerprogrammes berechnet werden. Erst dadurch wird es möglich, die Teststreifen auszuwerten und eine nicht mehr vorhandene Anschmutzungsfläche **2** zumindest rechnerisch zu ermitteln, ohne eine Maske oder ähnliches zur Hilfe zu nehmen. Alternativ kann die Kennzeichnung eine Rahmung sein, um die Testanschmutzungsgesamtfläche nach der Reinigung sichtbar zu machen. Bedeutsam jedoch ist, dass die nicht mehr vorhandenen Anschmutzungsflächenareale sich nur durch ein Softwareprogramm berechnen lassen.

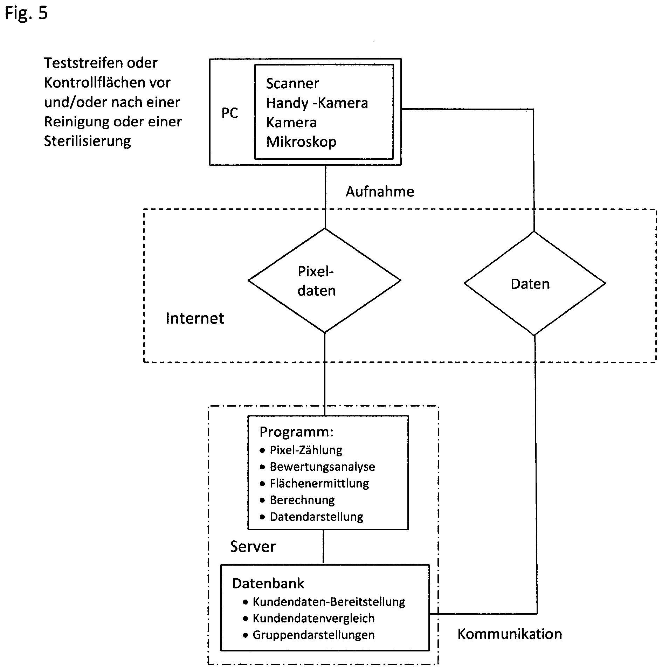

Figur 5. zeigt eine schematische Darstellung einer Analyse und Übertragung von Pixel-Daten. Die mit einem Scanner oder einer Kamera aufgenommenen Teststreifenbilder und/oder Bildpunkte (Pixel) werden über das Internet vorzugsweise mit einem Zuordnungscode (z. B. einen Nutzercode als Zugangsberechtigung) an einen Server übermittelt. Das hier vorhandene Programm zählt die Pixel, nimmt eine Bewertungsanalyse der Pixel vor und/oder berechnet hieraus eine Fläche. Die Daten können anschließend als Tabelle, Diagramm oder als grafische Darstellung den jeweiligen Übermittlungscode oder einer Datengruppe zugeordnet werden. Das Computerprogramm wiederum ist erfindungsgemäß in der Lage, die ermittelten Daten mit einem größeren Datenpool zu vergleichen. Damit hat der Nutzer die Möglichkeit seine Daten mit anderen gleich oder ähnlich erhobenen Daten zu vergleichen. Das wiederum verbessert die Einordnung der Reinigungsleistung, wodurch die Sicherheit erhöht wird. Alternativ kann das Serverprogramm auch bereits auf dem PC oder dem Handy (z. B. als App) installiert sein und die Daten ohne eine Übermittlung auswerten. Ziel ist es jedoch, immer die noch ermittelbare Restkontamination in Darstellung von Bildpunkten und/oder über eine Flächenberechnung quantitativ zu erfassen und soweit möglich auch qualitativ einzuordnen.

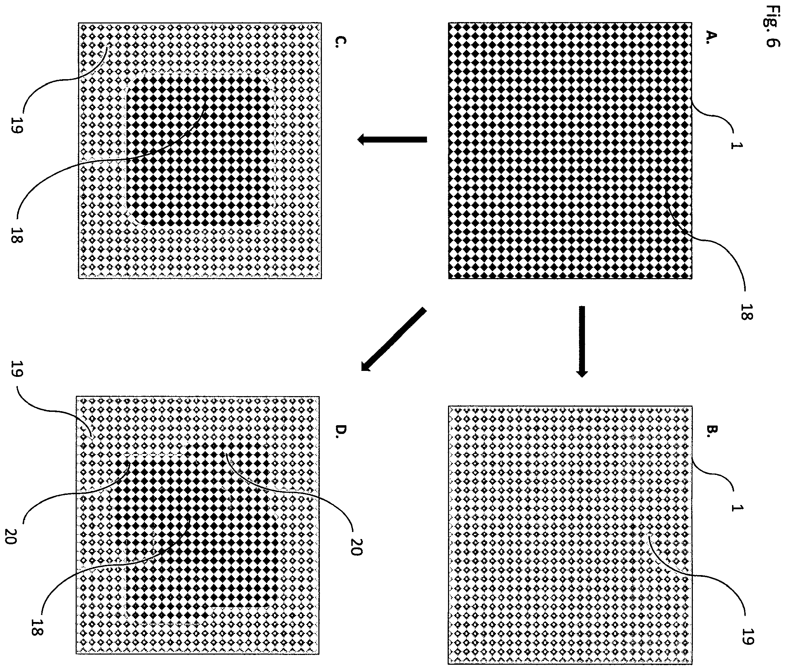

Figur 6. Zeigt eine Testfläche zur Bestimmung der Sterilisationsleistung. In der Abbildung **A.** ist ein Testpapier **1** mit einer z. B. ganzflächigen temperatursensiblen Farbmarkierung **18** eingesetzt (entspricht z. B. einem Bowie Dick Testpapier). Die gezeigte Farbfläche kann dabei ein Karomuster oder jede andersgeartete Farbmarkierung oder ein Farbmuster aufweisen. Abbildung **B.** zeigt einen vollständig umgewandeltes Testpapier **1** bei der die Ursprungsfarbe in eine andersfarbige Farbfläche **19** umgewandelt wurde. Abbildung **C.** zeigt eine nicht vollständig umgewandelte Farbmarkierungsfläche. Die äußere Fläche **19** ist umgewandelt, da hier der Dampf eingewirkt hat. Hingegen zeigt der mittlere Bereich **18,** das der Dampf hier nicht oder nur gering eingewirkt hat und kein Farbumschlag erfolgt ist. Abbilden **D.** zeigt ebenfalls eine inhomogene nicht vollständig umgewandelte farbige Flächenareale **20** mit unterschiedlichen Dampfeinwirkungsgrad. Diese können je nach Dampfdurchdringung statistisch verteilt sein oder jedwedes Muster oder eine Farbänderung aufweisen. Mit dem Auge lassen sich derartig geringen farbigen Änderungen eines Flächenmusters **20** oder einer Farbfläche nicht genau quantitativ und qualitativ bestimmen. Erst mit optischen Methoden ist eine genauere Aussage möglich. Durch einen groß angelegten Datenabgleich von vielen Untersuchungen, werden die so ermittelten Ergebnisse immer genauer und es lassen sich kleinste Unterschiede im Testsystem erkennen und hieraus wiederum Schlüsse für den Sterilisationserfolg ziehen als es derzeit möglich ist. Ferner ermöglich eine elektronische Datenerhebung die nachträgliche und visualisierte Darstellung vorhandener Flächenmuster **20** z. B. durch Rahmung und erleichtert damit zumindest die visuelle Bewertung.

Zeichenerklärung

1. Teststreifen, Kontrollkörper, Kontrollflächen oder Testpapiere mit Farbdruck
2. Filmische Anschmutzungsfläche gesamt, filmische Anschmutzungsmatrix oder eine Fläche die Stoffe enthält

3. Fläche ohne Anschmutzung

4. Nicht abwaschbare Kennzeichnung, Marker oder Markierung

5. Geschützte Anschmutzungsfläche (sichtbar nach der Reinigung)

6. Scannerglas oder farbiger Hintergrund

7. Detektierbare Bildpunkte (Pixel) oder Bildareale eines oder mehrerer hier vorhandenen Stoffe oder einer Restanschmutzung oder von Farbresten nach erfolgter Reinigung oder Sterilisation

8. Restanschmutzung im abgedeckten bzw. gesicherten Anschmutzungsbereich 5

9. Über- oder abgedeckte Anschmutzungsfläche oder Spaltbereich (zugänglich für Reinigungslösung

10. Barcode, QR-Code oder Zahlencode

11. Teststreifenecken

12. Beleuchtungseinrichtung z. B. eines Scanners

13. Scanner-Bewegungsarm ober Abtastarm

14. Farbmolekül gebunden (z. B. elektrostatische Wechselwirkung oder ionisch)

15. Farbmolekül gebunden durch van der Waals Wechselwirkung (schwache Wechselwirkung)

16. Stoffmolekül ohne gebundenen Farbstoffmolekül

17. Stoffmolekül mit einem gebundenen Farbstoffmolekül (Farbmolekülkomplex)

18. Temperatursensible Farbmarkierung oder Farbfläche (nicht umgewandelt)

19. vollständig umgewandelte Farbmarkierung oder Farbfläche

20. Nicht vollständig umgewandelte Farbmarkierung oder Farbfläche

21. Einkerbungen

## Patentansprüche

1. Analyseverfahren, für die optische Bestimmung von Teststreifen- oder von Kontrollflächen (1) nach einer erfolgten Behandlung mit einem Computerprogramm, wobei zumindest nach einer Reinigungs- und/oder Sterilisation der Teststreifen- oder der Kontrollflächen (1) die vorhandenen Stoffe auf den Oberflächen (2) der Teststreifen oder der Kontrollflächen im ersten Schritt mit optischen Methoden als Pixel (7) aufgenommen werden *und es folgt eine Datenübermittlung an einem Server über das Internet und das Softwareprogramm zur Pixelanalyse auf dem Server nimmt zumindest eine Pixelzählung vor und bewertet die Pixel nach Intensität,* um im zweiten Schritt mit Unterstützung von Markern (4, 10), Zeichen (5), Rahmen, Barcodes, Ecken (11) oder Flächen (1, 18, 19, 20) die Pixel vor der Behandlung zu berechnen oder zu rekonstruieren oder diese virtuell zu erzeugen oder zu konstruieren, um in einem dritten Schritt zumindest durch die Abnahme der Pixelmenge oder durch die Pixeländerung einer Oberflächenfarbe und/oder eines Oberflächenmusters nach einer erfolgten Behandlung den Reinigungs- oder den Sterilisationserfolg *einzelner Analyseergebnisse im Vergleich* zu einem *gesammelten Datenpool* zu ermitteln.

2. Analyseverfahren nach Anspruch 1, **dadurch gekennzeichnet, dass** bei der Auswertung der Flächen (1, 2, 5, 18, 19, 20) oder des geometrischen Farbmusters oder der Farbbilder die Flächenbereiche in der eine Farbänderung auf der Fläche nach der Reinigung oder nach der Sterilisierung oder nach einer Dekontamination erfolgt, bewertet, herausgehoben oder anzeigt werden, um damit zumindest eine Temperaturbehandlung und/oder eine pH-Wertänderung anzuzeigen und/oder um Partikel oder Reststoffe auf einer Fläche zu bestimmen.

3. Analyseverfahren, nach Anspruch 1 und 2, **dadurch gekennzeichnet**, das die Teststreifen oder Kontrollflächen ungeordnet oder verstreut liegend mit optischen Methoden oder mit Bildaufnahmegeräte aufgenommen werden, um anschließend in den berechneten Anschmutzungsflächen (2) zumindest die noch farbigen und auflösbaren Pixel (7) zu analysieren, wobei ein Pixel (7) näherungsweise eine Stoffmenge und/oder eine Farbmenge oder zumindest eine Farbintensität auf einer Oberfläche nach einer erfolgten Reinigung charakterisiert und hierrüber die Reinigungsleistung bestimmt wird.

4. Analyseverfahren nach einem der Ansprüche 1 bis 3, **dadurch gekennzeichnet, dass** eine Sterilisation erfolgt ist und die Teststreifen oder Kontrollflächen (1) eine Farbänderung erfahren haben, um das mit einem Computerprogramm berechnete Pixel-Gesamtflächenmuster einer Farbe vor der Behandlung mit einer nach der Behandlung erzeugten Pixelmenge einer Farbe und/oder eines Flächenmusters zu vergleichen, um zumindest durch die Farbintensität und/oder über die Farbänderung der aufgenommenen Pixel (7) den Sterilisationserfolg zu bestimmen.

5. Analyseverfahren nach einem der Ansprüche 1 bis 4, **dadurch gekennzeichnet, dass** die sich ändernde Flächen (2, 18) dem Computerprogramm bekannt sind und/oder bei der Analyse durch Markierungen (4) oder Einkerbungen (21) oder Kennzeichnungen (10) zusätzlich eine Behandlungsrichtung erkennbar ist oder diese mit Hilfe zumindest von Eichbildern oder Eichflächen mit einem Computer- oder Softwareprogramm zu berechnen oder virtuell zu erzeugen, um mit diesen Daten die aufgenommenen Pixeldaten (7) nach einer Behandlung zu vergleichen

6. Analyseverfahren nach einem der Ansprüche 1 bis 5, **dadurch gekennzeichnet, dass** die Flächenausgestaltung, die Farbintensität und die Farbe einer Stoffmenge oder eines Farbmarkers auf einer Oberfläche (1, 2) in der die Farbmoleküle (14) eingebunden und/oder mit dieser molekular vernetzt sind, aufgenommen wird und anschließend ein Computer- oder Softwareprogram zumindest die Bilddaten in Pixel (7) überführt, wobei mindestens 1 Bytes pro Pixel einen Speicherplatz belegt und zumindest ein Farbraummodel Anwendung findet.

7. Analyseverfahren nach einem der Ansprüche 1 bis 6, **dadurch gekennzeichnet, dass** in der filmischen Anschmutzungsfläche (2) Farbmoleküle an Anschmutzungsmoleküle chemisch gebunden sind und/oder die Farbmoleküle durch schwache nicht-kovalente Wechselwirkungen in der filmischen Beschichtungsmatrix festgehalten werden und sich über eine Eichung oder über Labormethoden zumindest für einen Pixel (7) oder eine Pixelmenge eine Farbmolekülmenge und/oder eine Stoffmenge zuordenbar ist und sich hieraus ein Umrechnungsfaktor zur Berechnung der Restanschmutzung ergibt.

8. Analyseverfahren nach einem der Ansprüche 1 bis 6, **dadurch gekennzeichnet, dass** mit einem Handy, einer Kameras, einem Mikroskope oder einem Scanner unterschiedliche und mehrere Teststreifen oder Kontrollflächen aufgenommen werden, um vor und/oder nach einem Reinigungs- und/oder Sterilisationsverfahren über ein Programm zumindest über die ermittelten Pixel (7) eine Restanschmutzungsmenge oder eine Farbänderung zu bestimmen und die ermittelten Daten über das Internet auszutauschen und mit einem größeren Datenpool zu vergleichen und/oder in Datengruppen abzubilden.

9. Analyseverfahren nach einem der voranstehenden Ansprüche, **dadurch gekennzeichnet, dass** die Teststreifenoberfläche reinigungsstabile Markierungsstriche (4) eine Rahmung, Punkte, Dreiecke und/oder Barcodes (10) oder Teststreifenecken (11) besitzt oder die Teststreifenoberfläche mit Hilfe eines farbigen Hintergrunds sichtbar ist, um die aufgenommenen Bilddaten mit einem Programm zu berechnen und zu bewerten, wobei das Programm zur Auswertung auf einem Server liegt, um weitere Nutzerdaten für die Auswertung zu nutzen.

10. Analyseverfahren nach einem der voranstehenden Ansprüche, **dadurch gekennzeichnet, dass** eine Zusatzbeleuchtung und/oder eine Abstandsvorrichtung zur besseren Pixelbestimmung und/oder eine Teststreifen- oder eine Kontrollfläche mit einer Musterfarbe oder eine Musterfarbtafel zur Eichung eingesetzt ist.

11. Analyseverfahren nach einem der voranstehenden Ansprüche, **dadurch gekennzeichnet, dass** die Markierung (4, 10) fälschungssichere Informationen wie fortlaufende Barcods (10), QR-Codes oder Nummerncodes aufweisen oder Farbcodes oder Farbflächen (5) besitzen und/oder diese bei einer Behandlung einen irreversiblen Farbumschlag erfahren.

12. Analyseverfahren nach einem der voranstehenden Ansprüche, **dadurch gekennzeichnet, dass** im Programm Algorithmen oder algorithmische Formel eingesetzt werden und der gesammelte Datenpool die Auswertung oder die Interpretation der ermittelten Bildpunkte unterstützt und/oder die ermittelte Farbintensität, der Farbton oder die Helligkeit der Bildpunkte und/oder deren Verteilung auf einer Oberfläche in die Analyse einbezogen wird.

13. Analyseverfahren nach einem der voranstehenden Ansprüche, **dadurch gekennzeichnet, dass** der gesammelter Datenpool oder die Auswertung oder deren Interpretation der ermittelten Bildpunkte mit einem Maschinen Learning Verfahren bewertet werden und hierüber zumindest mit einer statischen Methode erfasst und/oder Flächenbereiche sichtbar herausgehoben und/oder in Zonen einteilt werden, in denen Änderungen erfolgt sind.

14. *Analyseverfahren nach einem der Ansprüche 1 bis 13*, insbesondere für die Bestimmung von Restanschmutzungsmengen auf Teststreifen zur Bestimmung der Reinigungsleistung, **dadurch gekennzeichnet, dass** eine farbige filmische Anschmutzungsfläche (2) oder filmische farbige Flächenareale eingesetzt sind, die ein Gemisch aus 2 - 12 % Emulgator und Biostoffe oder ein Gemisch aus 2 - 12 % Emulgator und geronnenes Blut oder eine Kombination aus diesen enthält.

15. Analyseverfahren nach Anspruch 14, **dadurch gekennzeichnet, dass** das Gemisch aus 5 - 35 % Lipide, 2 - 20 % Proteine, 0,2 - 2 % Kohlenhydrate, 0,1 - 1 % Mineralstoffe und Farbstoffe enthält und diese mit Wasser und/oder mit Blut vermischt sind und bei dem zumindest ein Emulgator natürlichen Ursprungs oder ein Emulgator-Gemisch vorhanden ist.

**16.** *Analyseverfahren* nach Anspruch 15, **dadurch gekennzeichnet, dass** chemisch unbedenkliche Farbstoffe aus Lebensmitteln und/oder Farbpigmente zur Färbung eingesetzt sind.

**Claims**

**1.** Analysis method for the optical determination of test strip or control surfaces (1) after treatment with a computer program, wherein at least after cleaning and/or sterilization of the test strip or control surfaces (1), the substances present on the surfaces (2) of the test strips or control surfaces are recorded as pixels (7) in the first step using optical methods, followed by data transmission to a server via the Internet and the software program for pixel analysis on the server carries out at least one pixel count and evaluates the pixels according to intensity, in order to calculate or reconstruct the pixels before the treatment with the support of markers (4, 10), characters (5), frames, barcodes, corners (11) or surfaces (1, 18, 19, 20) or to generate in a second step or construct these virtually in order to determine the cleaning or sterilization success of individual analysis results in a third step at least by the decrease in the pixel quantity or by the pixel change of a surface colour and/or a surface pattern after a treatment has taken place in comparison with a collected data pool.

**2.** Analysis method according to claim 1, **characterised in that** during the evaluation of the surfaces (1, 2, 5, 18, 19, 20) or the geometric colour pattern or the colour images, the surface areas in which a colour change occurs on the surface after cleaning or after sterilisation or after decontamination are evaluated, highlighted or displayed in order to indicate at least a temperature treatment and/or a pH value change and/or to determine particles or residual substances on a surface.

**3.** Analysis method according to claims 1 and 2, **characterised in that** the test strips or control surfaces are recorded in a disordered or scattered manner using optical methods or image recording devices, in order to subsequently analyse at least the still coloured and resolvable pixels (7) in the calculated soiling areas (2), wherein a pixel (7) approximately characterises a substance quantity and/or a colour quantity or at least a colour intensity on a surface after cleaning has taken place and the cleaning performance is determined by means of this.

**4.** Analysis method according to one of claims 1 to 3, **characterised in that** sterilisation has taken place and the test strips or control surfaces (1) have undergone a colour change in order to compare the total pixel area pattern of a colour calculated with a computer program before the treatment with a pixel quantity of a colour and/or an area pattern generated after the treatment in order to determine the sterilisation success at least by the colour intensity and/or via the colour change of the recorded pixels (7).

**5.** Analysis method according to one of claims 1 to 4, **characterised in that** the changing surfaces (2, 18) are known to the computer program and/or a treatment direction is additionally recognisable during the analysis by means of markings (4) or notches (21) or markings (10), or this can be calculated or virtually generated with the aid of at least calibration images or calibration surfaces with a computer or software program in order to compare the recorded pixel data (7) after a treatment with this data

**6.** Analysis method according to one of claims 1 to 5, **characterised in that** the surface configuration, the colour intensity and the colour of a substance quantity or a colour marker on a surface (1, 2) in which the colour molecules (14) are incorporated and/or molecularly cross-linked with the latter is recorded and then a computer or software program converts at least the image data into pixels (7), whereby at least 1 byte per pixel occupies a memory location and at least one colour space model is applied.

**7.** Analysis method according to one of claims 1 to 6, **characterised in that** dye molecules are chemically bound to soiling molecules in the filmic soiling area (2) and/or the dye molecules are retained in the filmic coating matrix by weak noncovalent interactions and can be assigned a colour molecule quantity and/or a substance quantity via a calibration or via laboratory methods for at least one pixel (7) or a pixel quantity and a conversion factor for calculating the residual soiling results from this.

**8.** Analysis method according to one of claims 1 to 6, **characterised in that** different and several test strips or control surfaces are recorded with a mobile phone, a camera, a microscope or a scanner in order to determine a residual soiling quantity or a colour change before and/or after a cleaning and/or sterilisation process via a program at least via the determined pixels (7) and to exchange the determined data via the Internet and to compare it with a larger data pool and/or to map it in data groups.

9. Analysis method according to one of the preceding claims, **characterised in that** the test strip surface has marking lines immutable to effects of cleaning (4), a frame, dots, triangles and/or barcodes (10) or test strip corners (11) or the test strip surface is visible with the aid of a coloured background in order to calculate and evaluate the recorded image data with a program, whereby the program for evaluation is located on a server in order to use further user data for the evaluation.

10. Analysis method according to one of the preceding claims, **characterised in that** an additional illumination and/or a spacing device for better pixel determination and/or a test strip or a control surface with a sample colour or a sample colour chart for calibration is used.

11. Analysis method according to one of the preceding claims, **characterised in that** the markings (4, 10) have tamper-proof information such as consecutive barcodes (10), QR codes or number codes or have colour codes or coloured areas (5) and/or these undergo an irreversible colour change during treatment.

12. Analysis method according to one of the preceding claims, **characterised in that** algorithms or algorithmic formulae are used in the program and the collected data pool supports the evaluation or the interpretation of the determined pixels and/or the determined colour intensity, hue or brightness of the pixels and/or their distribution on a surface is included in the analysis.

13. Analysis method according to one of the preceding claims, **characterised in that** the collected data pool or the evaluation or interpretation thereof of the determined pixels is evaluated using a machine learning method and is recorded using at least a static method and/or surface areas are visibly highlighted and/or divided into zones in which changes have occurred.

14. Analysis method according to one of claims 1 to 13, in particular for the determination of residual soiling quantities on test strips for determining the cleaning performance, **characterised in that** a coloured filmic soiling surface (2) or filmic coloured surface areas are used, which contains a mixture of 2 - 12 % emulsifier and biological substances or a mixture of 2 - 12 % emulsifier and coagulated blood or a combination of these.

15. Analysis method according to claim 14, **characterised in that** the mixture contains 5 - 35 % lipids, 2 - 20 % proteins, 0.2 - 2 % carbohydrates, 0.1 - 1 % minerals and colouring agents and these are mixed with water and/or with blood and in which at least one emulsifier of natural origin or an emulsifier mixture is present.

16. Analysis method according to claim 15, **characterised in that** chemically harmless colouring agents from foodstuffs and/or colour pigments are used for colouring.

**Revendications**

1. Méthode d'analyse pour la détermination optique de surfaces de bandes de test ou de surfaces de contrôle (1) après un traitement effectué avec un programme informatique, dans le cadre duquel, après au moins un nettoyage et/ou une stérilisation des surfaces de bandes de test ou des surfaces de contrôle (1), les substances présentes sur les surfaces (2) des bandes de test ou celles de contrôle sont enregistrées dans la première étape avec des méthodes optiques sous forme de pixels (7), une transmission de données à un serveur par l'intermédiaire d'Internet doit alors suivre et le programme logiciel d'analyse de pixels sur le serveur effectue au moins un comptage de pixels et évalue ces derniers selon leur intensité, pour, dans une deuxième étape, les calculer ou les reconstruire les pixels avant le traitement ou les générer ou les construire virtuellement à l'aide de marqueurs (4, 10), de caractères (5), de cadres, de codes à barres, d'angles (11) ou de surfaces (1, 18, 19, 20), pour, dans une troisième étape, déterminer le succès du nettoyage ou de la stérilisation de résultats d'analyse individuels par rapport à un pool de données collectées, au moins par la diminution de la quantité de pixels ou par la modification des pixels d'une couleur de surface et/ou d'un motif de surface après un traitement effectué.

2. Méthode d'analyse selon la revendication 1, **caractérisée par** l'évaluation des surfaces (1, 2, 5, 18, 19, 20), du modèle de couleur géométrique ou des images en couleur; les zones dans lesquelles un changement de couleur se produit sur la surface après le nettoyage, la stérilisation ou une décontamination sont évaluées, mises en évidence ou indiquées, afin de mettre ainsi en évidence au moins un traitement thermique et/ou un changement de valeur de pH et/ou de déterminer des particules ou des substances résiduelles sur une surface.

**3.** Méthode d'analyse selon les revendications 1 et 2, **caractérisée par** l'enregistrement des bandes de test ou des surfaces de contrôle désordonnées ou dispersées avec des méthodes optiques ou des appareils d'enregistrement d'images, pour analyser ensuite dans les surfaces de salissure (2) calculées au moins les pixels (7) encore colorés et pouvant être résolus, un pixel (7) caractérisant approximativement une quantité de substance et/ou une quantité de couleur ou une intensité de couleur sur une surface après un nettoyage effectué, permettant ainsi de déterminer l'efficacité de ce nettoyage.

**4.** Méthode d'analyse selon l'une des revendications 1 à 3, **caractérisée par** une stérilisation effectuée et des bandes de test ou des surfaces de contrôle (1) qui ont subi un changement de couleur, afin de comparer le motif de la surface totale de pixels d'une couleur avant le traitement, calculé à l'aide d'un programme informatique, à une quantité de pixels d'une couleur et/ou d'un motif de surface généré après le traitement, dans le but de déterminer le succès de la stérilisation par l'intensité de la couleur et/ou par le biais du changement de couleur des pixels (7) enregistrés au moins.

**5.** Méthode d'analyse selon l'une des revendications 1 à 4, **caractérisée par** des surfaces (2, 18) changeantes connues du programme informatique et/ou par une direction de traitement reconnaissable lors de l'analyse en plus par des repères (4), des encoches (21) ou des marquages (10), ou par la possibilité de calculer ou de générer virtuellement celle-ci à l'aide au moins des images ou des surfaces d'étalonnage avec un programme informatique ou logiciel, afin de comparer les données de pixels (7) enregistrées après un traitement avec ces données

**6.** Méthode d'analyse selon l'une des revendications 1 à 5, **caractérisée par** l'enregistrement de la configuration de surface, de l'intensité de couleur et de la couleur d'une quantité de substance ou d'un marqueur de couleur sur une surface (1, 2) dans laquelle les molécules de couleur (14) sont intégrées et/ou réticulées moléculairement avec celle-ci ; un programme informatique ou logiciel convertit ensuite au moins les données d'image en pixels (7), avec au moins 1 octet par pixel occupant un emplacement de mémoire et au moins un modèle d'espace de couleur utilisé.

**7.** Méthode d'analyse selon l'une des revendications 1 à 6, **caractérisée**, dans la surface de salissure filmique (2), par des molécules de couleur liées chimiquement à des molécules de salissure et/ou des molécules de couleur retenues dans la matrice de revêtement filmique par des interactions non covalentes faibles, et par une quantité de molécules de couleur et/ou de substance qui peut être affectée au moins à un pixel (7) ou à une quantité de pixels par l'intermédiaire d'un étalonnage ou de méthodes de laboratoire, avec comme résultat un facteur de conversion pour le calcul de la salissure résiduelle.

**8.** Méthode d'analyse selon l'une des revendications 1 à 6, **caractérisée par** des bandes de test ou des surfaces de contrôle différentes et multiples enregistrées avec un téléphone portable, une caméra, un microscope ou un scanner, afin de définir par l'intermédiaire d'un programme, avant et/ou après un procédé de nettoyage et/ou de stérilisation au moins les pixels (7) déterminés, une quantité de salissure résiduelle ou un changement de couleur, et d'échanger les données obtenues par l'intermédiaire d'Internet et de les comparer à un pool de données plus grand et/ou de les représenter dans des groupes de données.

**9.** Méthode d'analyse selon l'une des revendications précédentes, **caractérisée par** une surface des bandes de test dotée de traits de marquage (4) stables au nettoyage, d'un encadrement, de points, de triangles et/ou de codes à barres (10) ou de bandes de test angulaires (11), ou par une surface de bandes de test visible à l'aide d'un fond coloré, afin de calculer et d'évaluer les données d'image enregistrées à l'aide d'un programme, celui-ci étant situé sur un serveur pour l'évaluation, afin d'utiliser d'autres données d'utilisateur pour cette même évaluation.

**10.** Méthode d'analyse selon l'une des revendications précédentes, **caractérisée par** l'utilisation d'un éclairage supplémentaire et/ou d'un dispositif d'écartement pour une meilleure détermination des pixels, d'une surface de bande de test, d'une surface de contrôle avec une couleur d'échantillon ou d'un tableau de couleurs d'échantillon pour l'étalonnage.

**11.** Méthode d'analyse selon l'une des revendications précédentes, **caractérisée par** un marquage (4, 10) présentant des informations infalsifiables tel que des codes à barres continus (10), des codes QR ou des codes numériques ou possédant des codes de couleurs ou des surfaces colorées (5) et/ou ceux-ci subissant un changement de couleur irréversible lors d'un traitement.

**12.** Méthode d'analyse selon l'une des revendications précédentes, **caractérisée par** l'utilisation d'algorithmes ou d'une formule algorithmique dans le programme et par un pool de données collecté qui assiste l'évaluation ou l'interprétation des pixels déterminés et/ou prend en compte dans l'analyse l'intensité de couleur, la teinte ou la luminosité des pixels et/ou leur répartition sur une surface déterminée.

13. Méthode d'analyse selon l'une des revendications précédentes, **caractérisée par** un pool de données collecté, une évaluation ou une interprétation des pixels déterminés évalués au moyen d'un procédé d'apprentissage automatique et saisis au moins avec une méthode statique et/ou des zones de surface mises en évidence de manière visible et/ou divisées par les zones dans lesquelles des modifications ont été effectuées.

14. Méthode d'analyse selon l'une des revendications 1 à 13, en particulier pour définir les quantités de salissures résiduelles sur des bandes de test pour la détermination de l'efficacité de nettoyage, **caractérisée par** l'utilisation d'une surface de salissure sous forme de film coloré (2) ou de zones de surface à film coloré, qui contient un mélange de 2 à 12 % d'émulsifiant et de substances biologiques ou un mélange de 2 à 12 % d'émulsifiant et de sang coagulé ou une combinaison de ceux-ci.

15. Méthode d'analyse selon la revendication 14, **caractérisée par** un mélange contenant de 5 à 35 % de lipides, de 2 à 20 % de protéines, de 0,2 à 2 % de glucides, de 0,1 à 1 % de substances minérales et de colorants, ceux-ci étant mélangés avec de l'eau et/ou avec du sang, et dans lequel au moins un émulsifiant d'origine naturelle ou un mélange d'émulsifiants est présent.

16. Méthode d'analyse selon la revendication 15, **caractérisée par** l'utilisation de colorants chimiquement inoffensifs issus de denrées alimentaires et/ou de pigments colorés pour la coloration.

Fig. 1

A.

B.

C.

Fig. 2

Fig. 3

Fig. 4

## Fig. 5

Teststreifen oder
Kontrollflächen vor
und/oder nach einer
Reinigung oder einer
Sterilisierung

PC | Scanner
Handy -Kamera
Kamera
Mikroskop

Aufnahme

Pixel-
daten

Daten

Internet

**Programm:**
● Pixel-Zählung
● Bewertungsanalyse
● Flächenermittlung
● Berechnung
● Datendarstellung

Server

**Datenbank**
● Kundendaten-Bereitstellung
● Kundendatenvergleich
● Gruppendarstellungen

Kommunikation

20

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1970698 A2
- DE 102010033016
- DE 102011108029
- DE 102013218448 A1
- WO 2015193664 A1
- US 20100217620 A1